# EUROPÄISCHE PATENTSCHRIFT

(11) **EP 1 449 940 B1**
(45) Veröffentlichungstag und Bekanntmachung des Hinweises auf die Patenterteilung: **24.06.2009**
(21) Anmeldenummer: 04002384.8
(22) Anmeldetag: 04.02.2004
(51) Int. Cl.: C23F 11/14, E21B 41/02, C10L 3/06, E21B 37/06, C07C 233/00

(54) **Korrosions- und Gashydratinhibitoren mit verbesserter Wasserlöslichkeit und erhöhter biologischer Abbaubarkeit**
Corrosion and gas hydrates inhibitors with improved water solubilty and biodegradabilty
Inhibiteurs de corrosion et de formation d'hydrates de gaz , à solubilité dans l'eau et biodégradabilité améliorées

(30) Priorität: 24.02.2003 DE 10307727
(43) Veröffentlichungstag der Anmeldung: 25.08.2004
(73) Patentinhaber: Clariant Produkte (Deutschland) GmbH, 65929 Frankfurt am Main (DE)
(72) Erfinder: Dahlmann, Uwe, Dr., 69126 Heidelberg (DE); Feustel, Michael, Dr., 55278 Köngernheim (DE)

(56) Entgegenhaltungen:
- EP-A- 1 050 567
- EP-A- 1 069 147
- EP-A- 1 243 637

## Beschreibung

Die vorliegende Erfindung betrifft ein Additiv und ein Verfahren zur Korrosionsinhibierung und Gashydratinhibierung an und in Einrichtungen zur Förderung und Transport von Kohlenwasserstoffen in der Erdölförderung und - verarbeitung.

In technischen Prozessen, bei denen Metalle mit Wasser oder auch mit ÖI-Wasser-Zweiphasensystemen in Kontakt kommen, besteht die Gefahr der Korrosion. Die ist besonders ausgeprägt, wenn die wässrige Phase wie bei Erdölgewinnungs- und Verarbeitungsprozessen stark salzhaltig oder durch gelöste Sauergase, wie Kohlendioxid bzw. Schwefelwasserstoff, azid ist. Daher ist die Ausbeutung einer Lagerstätte und die Verarbeitung von Erdöl ohne spezielle Additive zum Schutz der eingesetzten Ausrüstungen nicht möglich.

Geeignete Korrosionsschutzmittel für die Erdölförderung und -verarbeitung sind zwar schon seit langem bekannt, jedoch aus Gründen des Umweltschutzes für Offshore-Anwendungen zukünftig inakzeptabel.

Als typische Korrosionsinhibitoren des Standes der Technik besitzen Amide, Amidoamine bzw. Imidazoline von Fettsäuren und Polyaminen eine äußerst gute Öllöslichkeit und sind somit in der korrosiven Wasserphase aufgrund schlechter Verteilungsgleichgewichte (Partitioning) nur in geringer Konzentration vorhanden. Demgemäss müssen diese Produkte trotz ihrer schlechten biologischen Abbaubarkeit in hoher Dosierung eingesetzt werden.

DE-A-199 30 683 beschreibt entsprechende Amidoamine/Imidazoline, die durch Umsetzung von Alkylpolyglykolethercarbonsäuren mit Polyaminen erhalten werden, und die aufgrund eines besseren Partitioning in geringeren Konzentrationen eingesetzt werden können.

Quartäre Alkylammoniumverbindungen (Quats) stellen alternative Korrosionsschutzmittel des Standes der Technik dar, die neben den korrosionsinhibierenden auch biostatische Eigenschaften besitzen können. Trotz einer verbesserten Wasserlöslichkeit zeigen die Quats, zum Beispiel im Vergleich zu den Imidazolinen, eine deutlich reduzierte Filmpersistenz und führen daher ebenfalls nur in höherer Dosierung zu einem effektiven Korrosionsschutz. Die starke Algentoxizität und die mäßige biologische Abbaubarkeit beschränken den Einsatz von Quats immer mehr auf ökologisch unsensible Anwendungsgebiete, z.B. Onshore.

EP-B-0 946 788 beschreibt ein Verfahren zum Schutz von Metalloberflächen gegenüber Korrosion unter Verwendungen von Esterquats, von denen eine gute biologische Abbaubarkeit und eine geringe Aquatoxizität offenbart wird.

EP-A-0 320 769 offenbart gegebenenfalls quaternierte Fettsäureester von oxalkylierten Alkylamino-alkylenaminen und deren Verwendung als Korrosionsinhibitor.

EP-B-0 212 265 beschreibt quartäre Polykondensate aus alkoxylierten Alkylaminen und Dicarbonsäuren und deren Verwendung als Korrosionsinhibitor und Spalter in Rohölen.

EP-B-0 446 616 beschreibt Ampholyte und Betaine auf Basis Fettsäure-Amidoalkylaminen, die unter den gegebenen Testbedingungen einen sehr guten Korrosionsschutz und eine wesentlich reduzierte Algentoxizität aufweisen.

EP-B-0 584 711 offenbart Ester, Amide bzw. Imide von Alkenylbernsteinsäuren mit Alkoxyalkylaminen und deren Metall- bzw. Ammoniumsalze als Emulgatoren und Korrosionsinhibitoren für Metallbearbeitungshilfsmittel. Die Verwendung von Alkenylbernsteinsäure-ester- bzw. amidoaminquats oder entsprechenden Betainen ist nicht beschrieben.

Gashydrate sind kristalline Einschlussverbindungen von Gasmolekülen in Wasser, die sich unter bestimmten Temperatur- und Druckverhältnissen (niedrige Temperatur und hoher Druck) bilden. Hierbei bilden die Wassermoleküle Käfigstrukturen um die entsprechenden Gasmoleküle aus. Das aus den Wassermolekülen gebildete Gittergerüst ist thermodynamisch instabil und wird erst durch die Einbindung von Gastmolekülen stabilisiert. Diese eisähnlichen Verbindungen können in Abhängigkeit von Druck und Gaszusammensetzung auch über den Gefrierpunkt von Wasser (bis über 25°C) hinaus existieren.

In der Erdöl- und Erdgasindustrie sind insbesondere die Gashydrate von großer Bedeutung, die sich aus Wasser und den Erdgasbestandteilen Methan, Ethan, Propan, Isobutan, n-Butan, Stickstoff, Kohlendioxid und Schwefelwasserstoff bilden. Insbesondere in der heutigen Erdgasförderung stellt die Existenz dieser Gashydrate ein großes Problem dar, besonders dann, wenn Nassgas oder Mehrphasengemische aus Wasser, Gas und Alkangemischen unter hohem Druck niedrigen Temperaturen ausgesetzt werden. Hier führt die Bildung der Gashydrate aufgrund ihrer Unlöslichkeit und kristallinen Struktur zur Blockierung verschiedenster Fördereinrichtungen, wie Pipelines, Ventilen oder Produktionseinrichtungen, in denen über längere Strecken bei niedrigeren Temperaturen Nassgas oder Mehrphasengemische transportiert werden, wie dies speziell in kälteren Regionen der Erde oder auf dem Meeresboden vorkommt.

Außerdem kann die Gashydratbildung auch beim Bohrvorgang zur Erschließung neuer Gas- oder Erdöllagerstätten bei entsprechenden Druck- und Temperaturverhältnissen zu Problemen führen, indem sich in den Bohrspülflüssigkeiten Gashydrate bilden.

Um solche Probleme zu vermeiden, kann die Gashydratbildung in Gaspipelines, beim Transport von Mehrphasengemischen oder in Bohrspülflüssigkeiten durch Einsatz von größeren Mengen (mehr als 10 Gew.-% bezüglich des Gewichts der Wasserphase) niederen Alkoholen, wie Methanol, Glykol, oder Diethylenglykol unterdrückt werden. Der Zusatz dieser Additive bewirkt, dass die thermodynamische Grenze der Gashydratbildung zu niedrigeren Temperaturen und höheren Drücken hin verlagert wird (thermodynamische Inhibierung). Durch den Zusatz dieser thermodynamischen Inhibitoren werden allerdings größere Sicherheitsprobleme (Flammpunkt und Toxizität der Alkohole), logistische Probleme (große Lagertanks, Recycling dieser Lösungsmittel) und dementsprechend hohe Kosten, speziell in der offshore-Förderung, verursacht.

Heute versucht man deshalb, thermodynamische Inhibitoren zu ersetzen, indem man bei Temperatur- und Druckbereichen, in denen sich Gashydrate bilden können, Additive in Mengen < 2 % zusetzt, die die Gashydratbildung entweder zeitlich hinauszögern (kinetische Inhibitoren) oder die Gashydratagglomerate klein und damit pumpbar halten, so dass diese durch die Pipeline transportiert werden können (sog. Agglomerat-Inhibitoren oder Anti-Agglomerates). Die dabei eingesetzten Inhibitoren behindern entweder die Keimbildung und/oder das Wachstum der Gashydratpartikel, oder modifizieren das Hydratwachstum derart, dass kleinere Hydratpartikel resultieren.

Als Gashydratinhibitoren wurden in der Patentliteratur neben den bekannten thermodynamischen Inhibitoren eine Vielzahl monomerer als auch polymerer Substanzklassen beschrieben, die kinetische Inhibitoren oder Agglomeratinhibitoren darstellen. Von besonderer Bedeutung sind hierbei Polymere mit Kohlenstoff-Backbone, die in den Seitengruppen sowohl cyclische (Pyrrolidon- oder Caprolactamreste) als auch acyclische Amidstrukturen enthalten.

EP-B-0 736 130 offenbart ein Verfahren zur Inhibierung von Gashydraten, welches die Zuführung einer Substanz der Formel 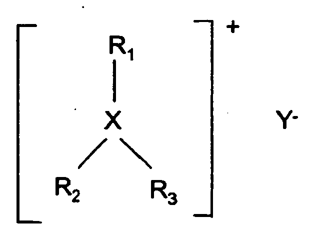 mit X = S, N - R₄ oder P - R₄, R₁, R₂ und R₃ = Alkyl mit mindestens 4 Kohlenstoffatomen, R₄ = H oder ein organischer Rest, und Y = Anion erfordert.

Umfasst werden also Verbindungen der Formel 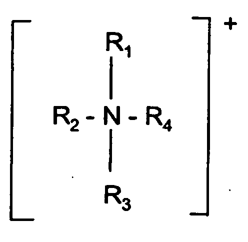 worin R₄ ein beliebiger Rest sein kann, die Reste R₁ bis R₃ aber Alkylreste mit mindestens 4 Kohlenstoffatomen darstellen müssen.

EP-B-0 824 631 offenbart ein Verfahren zur Inhibierung von Gashydraten, welches die Zuführung einer Substanz der Formel 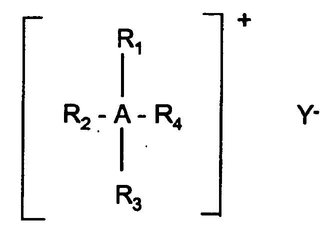 mit R₁, R₂ = gerade/verzweigte Alkylreste mit 4 oder 5 Kohlenstoffatomen, R₃, R₄ = organische Reste mit mindestens 8 Kohlenstoffatomen und A = Stickstoff oder Phosphor erfordert. Y⁻ ist ein Anion. Es müssen zwei der Reste R₁ bis R₄ gerade oder verzweigte Alkylreste mit 4 oder 5 Kohlenstoffatomen sein.

US-5 648 575 offenbart ein Verfahren zur Inhibierung von Gashydraten. Das Verfahren umfasst die Verwendung einer Verbindung der Formel 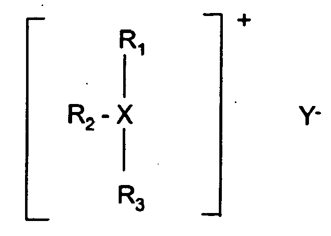 worin R¹, R² gerade oder verzweigte Alkylgruppen mit mindestens 4 Kohlenstoffatomen, R³ ein organischer Rest mit mindestens 4 Atomen, X Schwefel, NR⁴ oder PR⁴, R⁴ Wasserstoff oder ein organischer Rest, und Y ein Anion sind. Das Dokument offenbart nur solche Verbindungen, die mindestens zwei Alkylreste mit mindestens 4 Kohlenstoffatomen aufweisen.

US-6 025 302 offenbart Polyetheraminammoniumverbindungen als Gashydratinhibitoren, deren Ammoniumstickstoffatom neben der Polyetheraminkette 3 Alkylsubstituenten trägt.

WO-99/13197 offenbart Ammoniumverbindungen als Gashydratinhibitoren, die wenigstens eine mit Alkylcarbonsäuren veresterte Alkoxygruppe besitzen. Die Vorteile einer Verwendung von quartären Dicarbonsäurederivaten ist nicht offenbart.

WO-01/09082 offenbart ein Verfahren zur Herstellung von quartären Aminen, die jedoch keine Alkoxygruppen tragen, und deren Verwendung als Gashydratinhibitoren.

WO-00/078 706 offenbart quartäre Ammoniumverbindungen als Gashydratinhibitoren, die jedoch keine Carbonylreste tragen.

EP-B- 0 914 407 offenbart die Verwendung von trisubstituierten Aminoxiden als Gashydratinhibitoren.

DE-C-10114638 und DE-C-19920152 beschreiben die Verwendung von modifizierten Ethercarbonsäureamiden als Gashydratinhibitoren. Die Verwendung von quaternierten Ethercarbonsäurederivaten ist nicht offenbart.

Aufgabe der vorliegenden Erfindung war es, neue Korrosionsinhibitoren zu finden, die bei konstant gutem oder verbessertem Korrosionsschutz neben einer optimierten Wasserlöslichkeit, einer schnelleren Filmbildung und somit verbesserten Filmpersistenz auch eine verbesserte biologische Abbaubarkeit im Vergleich zu den Korrosionsinhibitoren des Standes der Technik bieten.

Aufgabe der vorliegenden Erfindung war es ferner, verbesserte Additive zu finden, die sowohl die Bildung von Gashydraten verlangsamen (kinetische Inhibitoren) als auch Gashydratagglomerate klein und pumpbar halten (Anti-Agglomerates), um so ein breites Anwendungsspektrum mit hohem Wirkpotential zu gewährleisten. Des weiteren sollten die derzeit verwendeten thermodynamischen Inhibitoren (Methanol und Glykole), die beträchtliche Sicherheitsprobleme und Logistikprobleme verursachen, ersetzt werden können.

Gashydratinhibitoren des Standes der Technik werden gewöhnlich mit Korrosionsinhibitoren co-additiviert, um Korrosion der Transport- und Fördereinrichtungen vorzubeugen. Aufgrund der häufig nicht unmittelbar gegebenen Verträglichkeit von Gashydratinhibitor und Korrosionsschutzmittel bei der Formulierung ergibt sich daraus für den Anwender ein zusätzlichen Aufwand. Ein wesentlicher Vorteil gegenüber dem Stand der Technik bestände, wenn eine Co-Additivierung mit Korrosionsinhibitoren nicht mehr zwingend erforderlich ist.

Es wurde nun überraschenderweise gefunden, dass quartäre Alkylaminoalkyl/alkoxy-ester bzw. quartäre Alkylamino-alkyl/alkoxy-amide von Dicarbonsäuren eine ausgezeichnete Wirkung als Korrosionsinhibitoren und Gashydratinhibitoren aufweisen, sowie eine verbesserte Filmpersistenz und gute biologische Abbaubarkeit zeigen.

Gegenstand der Erfindung ist somit die Verwendung von Verbindungen der Formel (1) 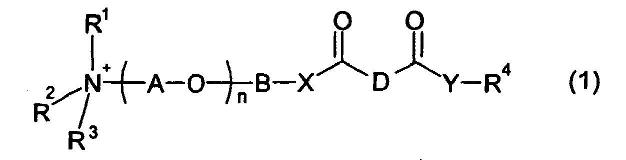 worin
- R¹, R²: unabhängig voneinander C₁- bis C₂₂-Alkyl, C₂- bis C₂₂-Alkenyl, C₆- bis C₃₀-Aryl- oder C₇- bis C₃₀-Alkylaryl,
- R³: C₁- bis C₂₂- Alkyl, C₂- bis C₂₂- Alkenyl, C₆- bis C₃₀-Aryl- oder C₇- bis C₃₀-Alkylaryl, -CHR⁵-COO- oder -O-,
- R⁴: M, Wasserstoff oder einen gegebenenfalls Heteroatome enthaltenden organischen Rest mit 1 bis 100 Kohlenstoffatomen,
- A: eine C₂- bis C₄-Alkylengruppe,
- B: eine C₁- bis C₁₀-Alkylengruppe,
- D: einen gegebenenfalls Heteroatome enthaltenden organischen Rest mit 1 bis 600 Kohlenstoffatomen,
- X, Y: unabhängig voneinander O oder NR⁶,
- R⁵, R⁶: unabhängig voneinander Wasserstoff, C₁- bis C₂₂-Alkyl, C₂- bis C₂₂-Alkenyl, C₆- bis C₃₀-Aryl- oder C₇- bis C₃₀-Alkylaryl, und
- M: ein Kation
- n: eine Zahl von 1 bis 30
bedeuten, als Korrosionsinhibitoren und Gashydratinhibitoren.

Ein weiterer Gegenstand der Erfindung ist ein Verfahren zur Inhibierung von Korrosion an Metalloberflächen, insbesondere von eisenhaltigen Metallen, indem einem korrosiven System, welches mit den Metalloberflächen in Kontakt steht, mindestens eine Verbindung der Formel (1) zugesetzt wird.

Ein weiterer Gegenstand der Erfindung ist ein Verfahren zur Inhibierung von Gashydraten, indem einem zur Bildung von Gashydraten neigenden System aus Wasser und Kohlenwasserstoffen mindestens eine Verbindung der Formel (1) zugesetzt wird.

Ein weiterer Gegenstand der Erfindung sind die Verbindungen der Formel (1).

Korrosive Systeme im Sinne dieser Erfindung sind bevorzugt flüssig/flüssig- bzw. flüssig/gasförmig-Mehrphasensysteme, bestehend aus Wasser und Kohlenwasserstoffen, die in freier und/oder gelöster Form korrosive Bestandteile, wie Salze und Säuren, enthalten. Die korrosiven Bestandteile können auch gasförmig sein, wie etwa Schwefelwasserstoff und Kohlendioxid.

Kohlenwasserstoffe im Sinne dieser Erfindung sind organische Verbindungen, die Bestandteile des Erdöls/Erdgases sind, und deren Folgeprodukte. Kohlenwasserstoffe im Sinne dieser Erfindung sind auch leichtflüchtige Kohlenwasserstoffe, wie beispielsweise Methan, Ethan, Propan, Butan. Für die Zwecke dieser Erfindung zählen dazu auch die weiteren gasförmigen Bestandteile des Erdöls/Erdgases, wie etwa Schwefelwasserstoff und Kohlendioxid.

A kann geradkettig oder verzweigt sein und steht vorzugsweise für eine Ethylenoder Propylengruppe, insbesondere eine Ethylengruppe. Bei den durch (A-O)ₙ bezeichneten Alkoxygruppen kann es sich auch um gemischte Alkoxygruppen handeln.

B kann geradkettig oder verzweigt sein und steht vorzugsweise für eine C₂- bis C₄-Alkylengruppe, insbesondere für eine Ethylen- oder Propylengruppe.

R¹ und R² stehen vorzugsweise unabhängig voneinander für eine Alkyl- oder Alkenylgruppe von 2 bis 14 Kohlenstoffatomen, insbesondere für solche Gruppen mit 3 bis 8 Kohlenstoffatomen und speziell für Butyl-Gruppen.

R³ steht vorzugsweise für eine Alkyl- oder Alkenylgruppe mit 1 bis 12 Kohlenstoffatomen, insbesondere mit 1 bis 4 Kohlenstoffatomen.

R⁵ und R⁶ stehen vorzugsweise für Wasserstoff.

n steht vorzugsweise für eine Zahl zwischen 1 und 10, besonders bevorzugt zwischen 2 und 6.

R⁴ kann ein beliebiger organischer Rest sein, der 1 bis 100 C-Atome enthält, und der gegebenenfalls Heteroatome enthalten kann. Enthält R⁴ Heteroatome, so handelt es sich vorzugsweise um Stickstoff- oder Sauerstoffatome oder beides, vorzugsweise um beides. Die Stickstoffatome können in quaternierter Form vorliegen.

R⁴ steht vorzugsweise für einen Rest der Formel (2) 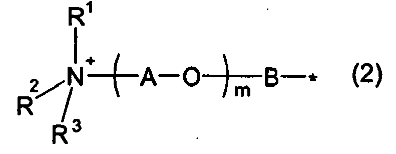 worin R¹, R², R³, A und B die bereits oben angegebenen Bedeutungen mit den jeweils oben für R¹, R², R³, A und B angegebenen Vorzugsbereichen haben. m steht unabhängig von n für eine Zahl zwischen 0 und 30, vorzugsweise zwischen 1 und 10, und besonders bevorzugt zwischen 2 und 6.

In einer weiteren bevorzugten Ausführungsform umfasst R⁴ Wasserstoff, der sowohl kovalent gebunden als auch dissoziert vorliegen kann.

D kann ein beliebiger bivalenter organischer Rest sein, der 1 bis 600 C-Atome enthält, und der gegebenenfalls Heteroatome enthalten kann. Die Verknüpfung der beiden Carbonylfunktionen mit D erfolgt vorzugsweise jeweils über eine freie Valenz einer Alkyl- bzw. Alkenyl-Gruppe an beliebiger Stelle von D.

Enthält D keine Heteroatome, so handelt es sich vorzugsweise um eine C₂- bis C₅₀-Alkylen- oder C₂- bis C₅₀-Alkenylen-Gruppe, die geradkettig oder verzweigt sein kann, und die sich von gesättigten und/oder ungesättigten Dicarbonsäuren, wie Adipinsäure, Bernsteinsäure, Maleinsäure bzw. Malonsäure, besonders bevorzugt von Fettsäuren, wie Dimerfettsäuren und Trimerfettsäuren, ableiten.

In einer weiteren besonders bevorzugten Ausführungsform steht D für C₆- bis C₅₀-Aryl- bzw. Alkylaryl-Reste, insbesondere für Reste, die sich von Benzendicarbonsäuren, besonders bevorzugt von Terephthalsäure (Benzen-1,4-dicarbonsäure) ableiten.

In einer weiteren bevorzugten Ausführungsform leitet sich D von substituierten Bernsteinsäurederivaten mit 10 bis 100 Kohlenstoffatomen ab. Der am Bernsteinsäurederivat gebundene Substituent steht vorzugsweise für eine C₂- bis C₉₀-Alkylen- oder C₂- bis C₉₀-Alkenylen-Gruppe, die als Oligomer von C₂- bis C₈-Alkenen erhältlich ist, und geradkettig oder verzweigt sein kann, und sich insbesondere von Ethylen, Propylen und Butylen ableitet.

Enthält D Heteroatome, so handelt es sich vorzugsweise um Stickstoff- oder Sauerstoffatome oder beides, vorzugsweise um beides. Die Stickstoffatome können in quaternierter Form vorliegen.

D steht vorzugsweise für einen Rest der Formel (3) 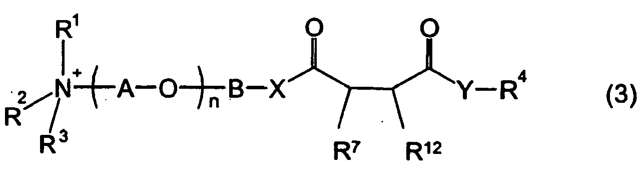 worin
R⁷ und R¹² entweder Wasserstoff oder einen C₂- bis C₁₀₀-Alkyl- oder C₂- bis C₁₀₀-Alkenyl-Rest, der als Oligomer von C₂- bis C₈-Alkenen erhältlich ist und geradkettig oder verzweigt sein kann, und insbesondere von Ethylen, Propylen und Butylen abgeleitet ist, bedeuten, mit der Maßgabe, dass genau einer der Reste R⁷ und R¹² für Wasserstoff steht. R¹, R², R³, R⁴, A, B, X, Y und n haben die bereits oben angegebenen Bedeutungen mit den jeweils oben für R¹, R², R³, R⁴, A, B, X, Y und n angegebenen Vorzugsbereichen.

Bei den bevorzugten Ausführungsformen von D gemäß Formel (4) erfolgt die Verknüpfung von D gemäß Formel (1) über jeweils eine Valenz eines Alkyl- bzw. Alkenylrests an beliebiger Stelle von R⁷ bzw. R¹².

M steht für ein ein- oder mehrwertiges Kation, vorzugsweise Metallion, besonders bevorzugt Alkali- oder Erdalkalimetallionen.

In einer weiteren bevorzugten Ausführungsform steht M für ein Ammoniumion der Formel N⁺R⁸R⁹R¹⁰R¹¹, worin R⁸, R⁹, R¹⁰ und R¹¹ unabhängig voneinander Wasserstoff oder einen beliebigen organischen Rest, der 1 bis 100 C-Atome enthält, und der gegebenenfalls Heteroatome enthalten kann, bedeuten. Enthalten R⁸, R⁹, R¹⁰ und/oder R¹¹ Heteroatome, so handelt es sich vorzugsweise um Stickstoff- oder Sauerstoffatome oder beides, vorzugsweise um beides. Die Stickstoffatome können in quaternierter Form vorliegen.

Steht R³ für C₁- bis C₂₂- Alkyl, C₂- bis C₂₂- Alkenyl bzw. C₆- bis C₃₀- Alkylaryl, so sind als Gegenionen für die Verbindungen gemäß Formel (1) bis (3) alle Anionen geeignet, die die Löslichkeit der Verbindungen der Formel (1) bis (3) in den organisch-wässrigen Mischphasen nicht beeinträchtigen. Solche Gegenionen sind beispielsweise Methylsulfationen (Methosulfat) oder Halogenidionen.

Steht R³ für Reste -CHR⁵-COO- bzw. -O-, so sind die Verbindungen der Formel (1) bis (3) Betaine bzw. Aminoxide und besitzen als innere Salze (Ampholyte) ein intramolekulares Gegenion.

Die erfindungsgemäßen Verbindungen können alleine oder in Kombination mit anderen bekannten Korrosionsinhibitoren und/oder Gashydratinhibitoren eingesetzt werden. Im allgemeinen wird man soviel des erfindungsgemäßen Korrosionsinhibitors und/oder Gashydratinhibitors einsetzen, dass man unter den gegebenen Bedingungen einen ausreichenden Korrosionsschutz und Schutz vor Gashydratbildung erhält.

Bevorzugte Einsatzkonzentrationen der Korrosionsinhibitoren bezogen auf die reinen erfindungsgemäßen Verbindungen sind 5 bis 5000 ppm, bevorzugt 10 bis 1000, insbesondere 15 bis 150 ppm.

Die Gashydratinhibitoren werden im allgemeinen in Mengen zwischen 0,01 und 5 Gew.-% der reinen erfindungsgemäßen Verbindungen bezogen auf die wässrige Phase, vorzugsweise zwischen 0,05 und 2 Gew.-% verwendet.

Besonders geeignet als Korrosionsinhibitoren und/oder Gashydratinhibitoren sind auch Mischungen der erfindungsgemäßen Produkte mit anderen Korrosionsinhibitoren und/oder Gashydratinhibitoren des Standes der Technik.

Besonders geeignet als Korrosionsinhibitoren und somit eine bevorzugte Ausführungsform dieser Erfindung sind Mischungen der Verbindungen gemäß Formel (1) bis (3), wie mit Amidoaminen und/oder Imidazolinen aus Fettsäuren und Polyaminen und deren Salzen, quartären Ammoniumsalzen, oxethylierten/oxpropylierten Aminen, Amphoglycinaten und -propionaten, Betainen oder Verbindungen beschrieben in DE-A-19930683.

Besonders geeignet als Gashydratinhibitoren und somit eine bevorzugte Ausführungsform dieser Erfindung sind Mischungen der Verbindungen gemäß Formel (1) mit einem oder mehreren Polymeren mit einem durch Polymerisation erhaltenen Kohlenstoff-Backbone und Amidbindungen in den Seitenketten. Hierzu zählen besonders Polymere wie Polyvinylpyrrolidon, Polyvinylcaprolactam, Polyiisopropylacrylamid, Polyacryloylpyrrolidin, Copolymere aus Vinylpyrrolidon und Vinylcaprolactam, Copolymere aus Vinylcaprolactam und N-Methyl-N-Vinylacetamid sowie Terpolymere von Vinylpyrrolidon, Vinylcaprolactam und weiteren anionischen, kationischen und neutralen Comonomeren mit vinylischer Doppelbindung wie 2-Dimethylaminoethylmethacrylat, 1-Olefinen, N-Alkylacrylamiden, N-Vinylacetamid, Acrylamid, Natrium-2-acrylamido-2-methyl-1-propansulfonat (AMPS) oder Acrylsäure. Weiterhin sind auch Mischungen mit Homo- und Copolymeren von N,N-Dialkylacrylamiden wie N-Acryloylpyrrolidin, N-Acryloylmorpholin und N-Acryloylpiperidin oder N-Alkylacrylamiden wie Isopropylacrylamid sowie Homo- als auch Copolymere von alkoxyalkylsubstituierte (Meth)Acrylsäureester geeignet. Ebenfalls sind Mischungen mit Alkylpolyglykosiden, Hydroxyethylcellulose, Carboxymethylcellulose sowie anderen ionischen oder nichtionischen Tensidmolekülen geeignet.

In einer bevorzugten Ausführungsform der Erfindung werden die erfindungsgemäßen Gashydratinhibitoren in Mischung mit Polymeren verwendet, die in WO-A-96/08672 offenbart sind. Es handelt sich bei diesen Polymeren um solche, die Struktureinheiten der Formel 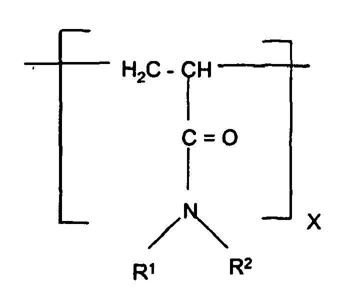 aufweisen, worin R¹ für eine Kohlenwasserstoffgruppe mit 1 bis 10 Kohlenstoffatomen und 0 bis 4 Heteroatomen, ausgewählt aus N, O und S, R² für eine Kohlenwasserstoffgruppe gleicher Definition wie R¹, und X für die durchschnittliche Zahl der sich wiederholenden Einheiten steht, wobei letztere so bemessen ist, daß das Polymer ein Molekulargewicht von 1000 bis 6 000 000 aufweist. Für die Verwendung im Umfang vorliegender Erfindung sind Polyisopropylacrylamide und Polyacryloylpyrrolidine besonders gut geeignet.

In einer weiteren bevorzugten Ausführungsform der Erfindung werden die erfindungsgemäßen Gashydratinhibitoren in Mischung mit Polymeren verwendet, die in WO-A-96/41785 offenbart sind. Dieses Dokument offenbart Gashydratinhibitoren, umfassend Struktureinheiten 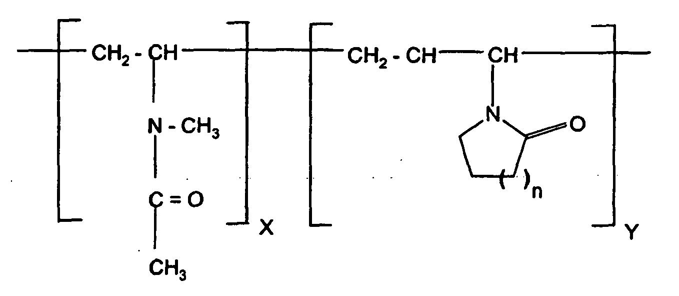 worin n eine Zahl von 1 bis 3 ist, und x und y die Zahl der repetitiven Einheiten darstellen, die so bemessen ist, daß das Molekulargewicht des Polymeren zwischen 1000 und 6 000 000 liegt. Für die Verwendung im Umfang vorliegender Erfindung sind Copolymere aus N-Vinylcaprolactam und N-Vinyl-N-Methylacetamid oder Vinylpyrrolidon besonders gut geeignet.

In einer weiteren bevorzugten Ausführungsform der Erfindung werden die erfindungsgemäßen Gashydratinhibitoren in Mischung mit anderen Polymeren verwendet, die in WO-A-94/12761 offenbart sind. Das Dokument offenbart Additive zur Verhinderung der Bildung von Gashydraten, welche Polymere, umfassend cyclische Substituenten mit 5 bis 7 Ringgliedern, enthalten. Für die Verwendung im Umfang vorliegender Erfindung sind insbesondere Polyvinylcaprolactam, Polyvinylpyrrolidon und Hydroxyethylcellulose geeignet.

Besonders geeignet sind auch Mischungen der erfindungsgemäßen Polymere mit Gashydratinhibitoren auf Maleinsäureanhydridbasis, wie sie in WO-A-97/6506 beschrieben sind, insbesondere mit Mono- und oder Diaminen umgesetzten Maleinsäureanhydridcopolymeren. Hierunter sind insbesondere modifizierte Vinylacetat-Maleinsäureanhydrid-Copolymere besonders bevorzugt.

In einer weiteren bevorzugten Ausführungsform der Erfindung werden die erfindungsgemäßen Gashydratinhibitoren in Mischung mit anderen Polymeren verwendet, die in DE-C-10059 816 offenbart sind. Das Dokument offenbart Additive zur Verhinderung der Bildung von Gashydraten, welche Polymere, umfassend Homo- als auch Copolymere, die alkoxyalkylsubstituierte (Meth)Acrylsäureester enthalten.

Werden Mischungen verwendet, so betragen die Konzentrationsverhältnisse zwischen den erfindungsgemäßen Gashydratinhibitoren und den zugemischten Komponenten von 90:10 bis 10:90 Gewichtsprozente, vorzugsweise werden Mischungen in den Verhältnissen 75:25 bis 25:75, und insbesondere von 60:40 bis 40:60 verwendet.

Die Verbindungen können, ebenso wie ihre Mischungen mit anderen Gashydratinhibitoren, bei der Erdöl- und Erdgasförderung oder der Bereitung von Bohrspülungen mittels gängiger Ausrüstung, wie Injektionspumpen o.ä. dem für die Hydratbildung anfälligen Mehrphasengemisch zugegeben werden, aufgrund der guten Löslichkeit der erfindungsgemäßen Polymere ist eine schnelle und gleichmäßige Verteilung des Inhibitors in der zur Hydratbildung neigenden Wasserphase bzw. der Kondensatphase gegeben.

Die erfindungsgemäßen Verbindungen können dadurch hergestellt werden, dass man alkoxylierte Alkylamine, Alkylaminoalkylenamine oder auch Alkylaminoalkyletheramine mit Dicarbonsäurederivaten zu den entsprechenden Mono- oder Dicarbonsäureestern bzw. Mono- oder Dicarbonsäureamiden, gegebenenfalls zu zyklischen Dicarbonsäureimiden, gemäß den Umsetzungsverhältnissen umsetzt. Anschließend wird mit geeigneten Alkylierungsmitteln quaterniert.

Die Herstellung von alkoxylierten Alkylaminen, Alkylaminoalkylenaminen oder auch Alkylaminoalkyletheraminen ist im Stand der Technik beschrieben.

Basis der verwendeten alkoxylierten Alkylamine sind Dialkylamine mit C₁- bis C₂₂-Alkylresten oder C₂- bis C₂₂-Alkenylresten, bevorzugt C₃- bis C₈-Dialkylamine. Geeignete Dialkylamine sind beispielsweise Di-n-butylamin, Diisobutylamin, Dipentylamin, Dihexylamin, Dioctylamin, Dicyclopentylamin, Dicyclohexylamin, Diphenylamin, Dibenzylamin.

Die Alkylamine werden im allgemeinen mit Ethylenoxid, Propylenoxid, Butylenoxid oder Mischungen verschiedener solcher Alkylenoxide umgesetzt, wobei Ethylenoxid oder Mischungen aus Ethylenoxid und Propylenoxid bevorzugt sind. Bezogen auf die Alkylamine werden 1-30 Mol Alkylenoxid beaufschlagt, bevorzugt 1-10 Mol, besonders bevorzugt 2-6 Mol.

Die Alkoxylierung erfolgt in Substanz, kann aber auch in Lösung durchgeführt werden. Geeignete Lösemittel für die Alkoxylierung sind inerte Ether wie Dioxan, Tetrahydrofuran, Glyme, Diglyme und MPEGs.

Im Allgemeinen wird die Alkoxylierung im ersten Reaktionsschritt unkatalysiert bis auf > 95 Gew.-% tert.-Stickstoff durchgeführt. Höheralkoxylierung erfolgt nach Zugabe basischer Verbindungen als Katalysatoren. Als basische Verbindungen können Erdalkali-/Alkalimetallhydroxide oder -alkoholate (Natriummethylat, Natriumethylat, Kalium-tert.-butylat) verwendet werden, bevorzugt sind aber Alkalimetallhydroxide, besonders Natriumhydroxid oder Kaliumhydroxid.

Basis der verwendeten Alkylaminoalkylenamine sind im wesentlichen Dialkylaminoalkylenamine mit C₁- bis C₂₂-Alkylresten oder C₂- bis C₂₂-Alkenylresten, bevorzugt tertiäre C₁- bis C₈-Dialkylamino-alkylenamine. Besonders geeignet sind beispielsweise N,N-Dibutylaminopropylamin, N,N-Diethylaminopropylamin, N,N-Dimethylamino-propylamin, N,N-Dimethylaminobutylamin, N,N-Dimethylaminohexylamin, N,N-Dimethylaminodecylamin, N,N-Dibutylaminoethylamin und N,N-Dimethylamino-2-hydroxypropylamin.

Basis der verwendeten Dicarbonsäurederivate sind freie Dicarbonsäuren, Dicarbonsäurediester, Dicarbonsäureanhydride und Dicarbonsäurehalogenide, bevorzugt Diester und Anhydride. Besonders geeignet sind Anhydride, beispielsweise Maleinsäureanhydrid, Bernsteinsäureanhydrid, Phthalsäureanhydrid und Alkenylbersteinsäureanhydride.

Die Herstellung von Alkenylbernsteinsäureanhydriden durch thermische oder katalysierte "En"-Reaktion ist im Stand der Technik beschrieben. Dabei werden Olefine, bevorzugt Olefine mit terminaler Doppelbindung, mit Maleinsäureanhydrid unter erhöhten Temperaturen umgesetzt. In Abhängigkeit von der Reaktionsführung, von der Art des verwendeten Olefins und vom verwendeten Molverhältnis werden Mono- und/oder Bisaddukte, gegebenenfalls Polyaddukte erhalten.

Die der Erfindung zugrundeliegenden Etherdicarbonsäuren können auf bekannten Wegen entweder durch Umsetzung der endständigen -CH₂OH-Funktion eines mit Alkylenoxid beaufschlagten Diols zur Carbonsäure durch Alkylierung mit Chloressigsäurederivaten nach der Williamsonschen Ether-Synthese oder durch Oxidation hergestellt werden.

Als geeignete Basisalkohole sind besonders bevorzugt C₂-C₃₀ Diole, wie Ethylenglykol, Propandiole und Butandiole, die mit Alkylenoxiden wie Ethylenoxid, Propylenoxid oder Butylenoxid oder Mischungen davon zu entsprechenden Glykolether umgesetzt werden, besonders bevorzugt ist Ethylenoxid. Die Produkte werden bevorzugt mit 1-30 mol Alkylenoxid, besonders bevorzugt 2-6 Mol Ethylenoxid umgesetzt.

Werden Mischungen der Alkylenoxide verwendet, können die resultierenden Alkylenglykol-alkylethern die Alkylenglykoleinheiten in statisch geregelter Abfolge und/oder als Blockoligomere enthalten.

Die Etherdicarbonsäure wird aus diesen Alkylenglykol-alkylethern durch Oxidation mit Luftsauerstoff unter Anwesenheit von Katalysatoren oder durch Oxidation mit Hypochlorit oder Chlorit mit und ohne Katalysatoren hergestellt.

Besonders bevorzugt ist aber die Alkylierung der Alkylenglykol-alkylethern mit Chloressigsäurederivaten, bevorzugt mit Natriumchloracetat und Natronlauge nach der Williamson-Methode. Die freie Carbonsäure wird aus dem alkalischen Alkylierungsgemisch durch Ansäuern mit Mineralsäure (Salz-, Schwefelsäure) und Erhitzen über den Trübungspunkt und Abtrennen der organischen Phase erhalten.

Die Dicarbonsäurederivate werden im allgemeinen mit den alkoxylierten Alkylaminen bzw. Alkylaminoalkylenaminen bei 60 - 240°C, bevorzugt bei 120 - 200°C derart umgesetzt, dass gegebenenfalls in Abhängigkeit vom verwendeten Dicarbonsäurederivat unter Eliminierung von Reaktionswasser oder von Alkohol eine vollständige Kondensation zum entsprechenden Mono- oder Dicarbonsäureestern bzw. Mono- oder Dicarbonsäureamiden, gegebenenfalls zu zyklischen Dicarbonsäureimiden, erfolgt. Der Umsetzungsgrad kann durch die Bestimmung der Säurezahl, Verseifungszahl und/oder durch die Bestimmung des Basen- und/oder Amidstickstoffs verfolgt werden.

Die Umsetzung erfolgt in Substanz, kann aber auch bevorzugt in Lösung durchgeführt werden. Insbesondere bei der Verwendung von Carbonsäuren ist die Verwendung von Lösemitteln erforderlich, wenn hohe Umsätze und niedrigere Säurezahlen von den resultierenden Umsetzungsprodukten angestrebt werden. Geeignete Lösemittel für die Herstellung sind organische Verbindungen, durch die das Reaktionswasser azeotrop entfernt wird. Insbesondere können aromatische Lösemittel oder Lösemittelgemische, oder Alkohole verwendet werden. Besonders bevorzugt ist 2-Ethylhexanol. Die Reaktionsführung erfolgt dann beim Siedepunkt des Azeotrops.

Bei der Herstellung von Dicarbonsäure-diamiden werden bevorzugt Dicarbonsäurediester und ein Überschuss des entsprechenden Amins verwendet, der mit dem frei werdenden Alkohol oder im Anschluss an die Umsetzung destillativ entfernt werden kann. Bei Verwendung von Dicarbonsäureanhydriden wird bevorzugt iterativ mit einem geeigneten Alkohol vollständig verestert und dann amidiert. Geeignete Alkohole sind z.B. Ethanol, Propanol, iso-Propanol oder 2-Ethylhexanol. Besonders bevorzugt ist 2-Ethylhexanol.

Gemäß dem Stand der Technik können die Veresterungen bzw. Amidierungen durch Zugabe geeigneter saurer Katalysatoren mit einem pKₐ-Wert von kleiner gleich 5, wie z.B. Mineralsäuren oder Sulfonsäuren, beschleunigt werden. Besonders bevorzugt sind Alkylstannansäuren.

Für die Herstellung der erfindungsgemäßen Verbindungen werden in einem anschließenden Reaktionsschritt die Alklyamino-alkyl/alkoxy-ester bzw. Alkylaminoalkyl/alkoxy-amide entsprechend quaterniert. Die Quaternierung kann durch entsprechende Alkylierungsmittel bei 50 bis 150 °C erfolgen. Geeignete Alkylierungsmittel stellen Alkylhalogenide und Alkylsulfate dar, bevorzugt Methylchlorid, Methyljodid, Butylbromid und Dimethylsulfat.

Für die Herstellung der erfindungsgemäßen Betaine wird mit einer Halogencarbonsäure und einer Base, bevorzugt Chloressigsäure und Natriumhydroxid umgesetzt. Dies kann geschehen, indem man die Alkylaminoamide mit 50 bis 125 Mol-% Halogencarbonsäure bei 40°C vorlegt und bei 40 bis 100°C durch einmalige oder portionierte Zugabe der Base und der zu 125 Mol-% verbleibenden Restmenge Halogencarbonsäure umsetzt.

Als basische Verbindungen können Erdalkali-/Alkalimetallhydroxide oder -alkoholate (Natriummethylat, Natriumethylat, Kalium-tert.-butylat) verwendet werden, bevorzugt sind aber Alkalimetallhydroxide, besonders Natriumhydroxid oder Kaliumhydroxid, insbesondere deren wässrigen Lösungen.

Die Herstellung der erfindungsgemäßen Aminoxide erfolgt nach bekannten Verfahren des Standes der Technik, vorzugsweise durch Oxidation der entsprechenden tertiären Amingruppe mit Peroxiden oder Persäuren, bevorzugt mit Wasserstoffperoxid.

Die Umsetzungen zu den quatären Alklyamino-alkyl/alkoxy-estern bzw. Alkylaminoalkyl/alkoxy-amiden erfolgen in Substanz, können aber auch bevorzugt in Lösung durchgeführt werden. Geeignete Lösemittel für die Herstellung der Quats, Betaine und Aminoxide sind inerte Alkohole wie Isopropanol, 2-Ethylhexanol oder inerte Ether wie Tetrahydrofuran, Glyme, Diglyme und MPEGs.

In Abhängigkeit von den gegebenen Anforderungen kann das verwendete Lösemittel im erfindungsgemäßen Produkt verbleiben oder muss destillativ entfernt werden.

### Beispiele:

### a) Allgemeine Vorschrift für die Herstellung der Alkylamino-alkyl/alkoxymonoester aus Dicarbonsäureanhydriden

In einer Rührapparatur mit Rückflusskühler wurden 0,3 mol des entsprechenden Anhydrids (gemäß Verseifungszahl) unter Stickstoffspülung vorgelegt und auf 60°C erwärmt. Dann wurden 0,3 mol des entsprechenden alkoxylierten Alkylendiamins (gemäß OH-Zahl) über 0,5 Stunde zugetropft, wobei sich die Reaktionsmischung auf ca. 70°C erwärmte. Die Reaktionsmischung wurde 5 h bei 60°C nachgerührt.

### Beispiel 1 (Dodecenyl-/Tetradecenylbernsteinsäure-N,N-dibutylamino-N-tri(ethoxy)ethylester)

Aus 84,2 g Dodecenyl-/Tetradecenylbernsteinsäureanhydrid (VZ = 399,9 mg KOH/g) und 72,9 g Dibutylamin + 3,7 EO (OHZ = 230,9 mg KOH/g) wurden ca. 155 g Dodecenyl-/Tetradecenylbernsteinsäure-N,N-dibutyl-amino-N-tri(ethoxy)ethylester mit SZ = 106,6 mg KOH/g und bas.-N = 2,24 % erhalten.

### Beispiel 2 (Tripropylenbernsteinsäure-N,N-dibutylamino-N-tri(ethoxy)ethylester)

Aus 74,1 g Tripropylenbernsteinsäureanhydrid (VZ = 454,5 mg KOH/g) und 72,9 g Dibutylamin + 3,7 EO (OHZ = 230,9 mg KOH/g) wurden ca. 145 g Tripropylenbernsteinsäure-N,N-dibutylamino-N-tri(ethoxy)ethylester mit SZ = 123,7 mg KOH/g und bas.-N = 2,39 % erhalten.

### Beispiel 3 (Tetrapropylenbernsteinsäure-N,N-dibutylamino-N-tri(ethoxy)ethylester)

Aus 87,8 g Tetrapropylenbernsteinsäureanhydrid (VZ = 383,3 mg KOH/g) und 72,9 g Dibutylamin + 3,7 EO (OHZ = 230,9 mg KOH/g) wurden ca. 160 g Tetrapropylenbernsteinsäure-N,N-dibutylamino-N-tri(ethoxy)ethylester mit SZ = 115,6 mg KOH/g und bas.-N = 2,18 % erhalten.

### Beispiel 4 (Pentapropylenbernsteinsäure-N,N-dibutylamino-N-tri(ethoxy)ethylester)

Aus 102,8 g Pentapropylenbernsteinsäureanhydrid (VZ = 327,6 mg KOH/g) und 72,9 g Dibutylamin + 3,7 EO (OHZ = 230,9 mg KOH/g) wurden ca. 175 g Pentapropylenbernsteinsäure-N,N-dibutylamino-N-tri(ethoxy)ethylester mit SZ = 100,5 mg KOH/g und bas.-N = 2,00 % erhalten.

### Beispiel 5 (Polyisobutenylbernsteinsäure-N,N-dibutylamino-N-tri(ethoxy)ethylester)

Aus 130,5 g Polyisobutenylbernsteinsäureanhydrid (auf Basis PIB 550; VZ = 257,9 mg KOH/g) und 72,9 g Dibutylamin+3,7EO (OHZ = 230,9 mg KOH/g) wurden ca. 200 g Polyisobutenylbernsteinsäure-N,N-dibutylamino-N-tri(ethoxy)ethylester mit SZ = 90,8 mg KOH/g und bas.-N = 1,74 % erhalten.

### b) Allgemeine Vorschrift für die Herstellung der der Alkylamino-alkyl/alkoxydieester aus Dicarbonsäureanhydriden

In einer Rührapparatur mit Destillationsbrücke wurden 0,3 mol des entsprechenden Anhydrids (gemäß Verseifungszahl) unter Stickstoffspülung vorgelegt und auf 60°C erwärmt. Dann wurden 0,6 mol des entsprechenden alkoxylierten Alkylendiamins (gemäß OH-Zahl) über 0,5 Stunde zugetropft, wobei sich die Reaktionsmischung auf ca. 70°C erwärmte. Die Reaktionsmischung wurde dann 0,5 h bei 60°C nachgerührt, anschließend auf 180°C erwärmt und bei dieser Temperatur 5 h Reaktionswasser abdestilliert. Abschließend wurde 2 h bei 200°C und 200 mbar das Reaktionswasser entfernt, bis eine Säurezahl (SZ) kleiner 10 mg KOH/g erreicht wurde.

### Beispiel 6 (Dodecenyl-/Tetradecenylbernsteinsäure-bis[N,N-dibutylamino-N-tri(ethoxy)ethylester])

Aus 84,2 g Dodecenyl-/Tetradecenylbernsteinsäureanhydrid (VZ = 399,9 mg KOH/g) und 145,8 g Dibutylamin + 3,7 EO (OHZ = 230,9 mg KOH/g) wurden ca. 220 g Dodecenyl-/Tetradecenylbernsteinsäure-bis[N,N-dibutyl-amino-N-tri(ethoxy)ethylester] mit SZ = 4,3 mg KOH/g, VZ = 120,1 mg KOH/g und bas.-N = 2,88 % erhalten.

### Beispiel 7 (Tripropylenbernsteinsäure-bis[N,N-dibutylamino-N-tri(ethoxy)ethylester])

Aus 74,1 g Tripropylenbernsteinsäureanhydrid (VZ = 454,5 mg KOH/g) und 145,8 g Dibutylamin + 3,7 EO (OHZ = 230,9 mg KOH/g) wurden ca. 210 g Tripropylenbernsteinsäure-bis[N,N-dibutylamino-N-tri(ethoxy)ethylester] mit SZ = 8,2 mg KOH/g, VZ = 127,7 mg KOH/g und bas.-N = 2,81 % erhalten.

### Beispiel 8 (Tetrapropylenbernsteinsäure-bis[N,N-dibutylamino-N-tri(ethoxy)ethylester])

Aus 87,8 g Tetrapropylenbernsteinsäureanhydrid (VZ = 383,3 mg KOH/g) und 145,8 g Dibutylamin + 3,7 EO (OHZ = 230,9 mg KOH/g) wurden ca. 225 g Tetrapropylenbernsteinsäure-bis[N,N-dibutylamino-N-tri(ethoxy)ethylester] mit SZ = 8,8 mg KOH/g, VZ = 115,4 mg KOH/g und bas.-N = 2,54 % erhalten.

### Beispiel 9 (Pentapropylenbernsteinsäure-bis[N,N-dibutylamino-N-tri(ethoxy)ethylester])

Aus 102,8 g Pentapropylenbernsteinsäureanhydrid (VZ = 327,6 mg KOH/g) und 145,8 g Dibutylamin + 3,7 EO (OHZ = 230,9 mg KOH/g) wurden ca. 240 g Pentapropylenbernsteinsäure-bis[N,N-dibutylamino-N-tri(ethoxy)ethylester] mit SZ = 7,7 mg KOH/g, VZ = 106,7 mg KOH/g und bas.-N = 2,75 % erhalten.

### Beispiel 1 0 (Polyisobutenylbernsteinsäure-bis[N,N-dibutylamino-N-tri(ethoxy)ethylester])

Aus 130,5 g Polyisobutenylbernsteinsäureanhydrid (auf Basis PIB 550; VZ = 257,9 mg KOH/g) und 145,8 g Dibutylamin + 3,7 EO (OHZ = 230,9 mg KOH/g) wurden ca. 270 g Polyisobutenylbernsteinsäure-bis[N,N-dibutylamino-N-tri(ethoxy)ethylester] mit SZ = 9,5 mg KOH/g, VZ = 94,9 mg KOH/g und bas.-N = 2,14 % erhalten.

### c) Allgemeine Vorschrift für die Herstellung der der Alkylamino-alkyl/alkoxydieester aus Etherdicarbonsäuren

In einer Rührapparatur mit Destillationsbrücke wurden 0,3 mol der entsprechenden Etherdicarbonsäuren (gemäß Verseifungszahl), 0,6 mol des entsprechenden alkoxylierten Alkylendiamins (gemäß OH-Zahl) und 0,5 Gew.-% p-Toluolsulfonsäure unter Stickstoffspülung vorgelegt und kontinuierlich auf 160°C erwärmt. Bei dieser Temperatur wurden über 4 h Reaktionswasser abdestilliert. Abschließend wurde die Destillation bei 200°C und 150 mbar fortgesetzt und das Reaktionswasser so lange entfernt, bis eine Säurezahl (SZ) kleiner 10 mg KOH/g erreicht wurde.

### Beispiel 11 (Polyglykoldicarbonsäure-bis[N,N-dibutylamino-N-tri(ethoxy)ethylester])

Aus 212,9 g Polyglykoldicarbonsäure (VZ = 158,1 mg KOH/g) und 145,8 g Dibutylamin + 3,7 EO (OHZ = 230,9 mg KOH/g) wurden ca. 340 g Polyglykoldicarbonsäure-bis[N,N-dibutylamino-N-tri(ethoxy)ethylester] mit SZ = 5,7 mg KOH/g, VZ = 80,7 mg KOH/g und bas.-N = 1,78 % erhalten.

### Beispiel 12 (3,6,9-Trioxa-undecandisäure-bis[N,N-dibutylamino-N-tri(ethoxy)ethylester])

Aus 78,8 g 3,6,9-Trioxa-undecandisäure (VZ = 427,3 mg KOH/g) und 145,8 g Dibutylamin + 3,7 EO (OHZ = 230,9 mg KOH/g) wurden ca. 205 g 3,6,9-Trioxaundecandisäure-bis[N,N-dibutylamino-N-tri(ethoxy)ethylester] mit SZ = 9,1 mg KOH/g, VZ = 132,9 mg KOH/g und bas.-N = 3,05 % erhalten.

### d) Allgemeine Vorschrift für die Quaternierung mit Dimethylsulfat

In einer Rührapparatur wurden 0,2 mol (nach bas.-N) des entsprechenden Amins unter Stickstoffspülung vorgelegt und auf 60°C erwärmt. Dazu wurden 0,19 mol Dimethylsulfat so zugetropft, dass die Reaktionstemperatur 80-90°C nicht übersteigt. Die Reaktionsmischung wurde anschließend 3 h bei 90°C nachgerührt. Nach dieser Vorschrift wurden die Verbindungen, beschrieben durch die Beispiele 1 bis 12 quaternisiert (Beispiele 13 bis 24, wie in Tabelle 1 bis 4 aufgeführt).

### Beispiel 25

Polyvinylcaprolactam mit MW 5000 g/mol werden im Verhältnis 1:1 mit dem durch Beispiel 13 beschriebenen Quat aus Beispiel 1 vermischt und in Butyldiglykol eingestellt.

### Beispiel 26

Polyvinylcaprolactam mit MW 5000 g/mol werden im Verhältnis 1:1 mit dem durch Beispiel 18 beschriebenen Quat aus Beispiel 6 vermischt und in Butyldiglykol eingestellt.

### Wirksamkeit der erfindungsgemäßen Verbindungen als Korrosionsinhibitoren

Die erfindungsgemäßen Verbindungen wurden als Korrosionsinhibitoren im Shell-Wheel-Test geprüft. Coupons aus C-Stahl (DIN 1.1203 mit 15 cm² Oberfläche) wurden in eine Salzwasser/Petroleum-Mischung (9:1,5 %ige NaCl-Lösung mit Essigsäure auf pH 3,5 gestellt) eingetaucht und bei einer Umlaufgeschwindigkeit von 40 rpm bei 70°C 24 Stunden diesem Medium ausgesetzt. Die Dosierung des Inhibitors betrug 50 ppm einer 40 % Lösung des Inhibitors. Die Schutzwerte wurden aus der Massenabnahme der Coupons, bezogen auf einen Blindwert, berechnet.

In den folgenden Tabellen bezeichnet "Vergleich 1" ein handelsübliches Rückstandsamin - Quat auf Basis Dicocosalkyl-dimethylammoniumchlorid und "Vergleich 2" ein handelsübliches Sojafettsäureamidopropyl-N,N-dimethylammonium-carboxymethyl-betain beschrieben durch EP-B-0 446 616 (Korrosionsinhibitoren des Standes der Technik).

**Tabelle 1: (SHELL-Wheel-Test)**

| Beispiel | Korrosionsinhibitor | ∅ Schutz % |
|---|---|---|
| Vergleich 1 | Standard-Quat | 36,0 |
| Vergleich 2 | Standard-Betain | 75,4 |
| 13 | Quat aus Beispiel 1 | 92,5 |
| 14 | Quat aus Beispiel 2 | 94,2 |
| 15 | Quat aus Beispiel 3 | 93,8 |
| 16 | Quat aus Beispiel 4 | 93,6 |
| 17 | Quat aus Beispiel 5 | 88,7 |
| 18 | Quat aus Beispiel 6 | 93,3 |
| 19 | Quat aus Beispiel 7 | 94,5 |
| 20 | Quat aus Beispiel 8 | 91,9 |
| 21 | Quat aus Beispiel 9 | 93,1 |
| 22 | Quat aus Beispiel 10 | 90,6 |

Die Produkte wurden außerdem im LPR-Test (Testbedingungen analog ASTM D 2776) geprüft.

**Tabelle 2: (LPR-Test)**

| Beispiel | Korrosionsinhibitor | Schutz nach [%] | | |
|---|---|---|---|---|
| | | 10 min | 30 min | 60 min |
| Vergleich 1 | Standard-Quat | 53,9 | 61,2 | 73,7 |
| Vergleich 2 | Standard-Betain | 45,9 | 59,2 | 64,3 |
| 13 | Quat aus Beispiel 1 | 79,6 | 86,1 | 87,6 |
| 14 | Quat aus Beispiel 2 | 79,1 | 85,1 | 87,3 |
| 15 | Quat aus Beispiel 3 | 84,6 | 89,6 | 91,2 |
| 16 | Quat aus Beispiel 4 | 81,8 | 87,3 | 89,7 |
| 17 | Quat aus Beispiel 5 | 74,1 | 80,0 | 83,5 |
| 18 | Quat aus Beispiel 6 | 78,9 | 85,0 | 86,7 |
| 19 | Quat aus Beispiel 7 | 81,6 | 86,4 | 88,2 |
| 20 | Quat aus Beispiel 8 | 76,2 | 82,8 | 85,9 |
| 21 | Quat aus Beispiel 9 | 77,3 | 81,6 | 83,2 |
| 22 | Quat aus Beispiel 10 | 68,9 | 74,2 | 79,8 |

Wie aus den obigen Testresultaten zu erkennen ist, weisen die erfindungsgemäßen Produkte sehr gute Korrosionsschutzeigenschaften bei niedriger Dosierung auf und übertreffen im wesentlichen die Wirksamkeit der Inhibitoren des Standes der Technik. Die Verbindungen besitzen aufgrund ihrer Ester- bzw. Amidstruktur eine bessere biologische Abbaubarkeit und können mit einer niedrigeren Dosierrate eingesetzt werden.

### Wirksamkeit der erfindungsgemäßen Verbindungen als Gashydratinhibitoren

Zur Untersuchung der inhibierenden Wirkung der erfindungsgemäßen Verbindungen wurde ein Stahl-Rührautoklav mit Temperatursteuerung, Druck- und Drehmomentaufnehmer mit 450 ml Innenvolumen benutzt. Für Untersuchungen zur kinetischen Inhibierung wurde der Autoklav mit destilliertem Wasser und Gas im Volumenverhältnis 20: 80 gefüllt, für Untersuchungen der Agglomeratinhibierung wurde zusätzlich Kondensat zugegeben. Abschließend wurde bei unterschiedlichen Drücken Erdgas aufgepresst.

Ausgehend von einer Anfangstemperatur von 17,5°C wurde innerhalb von 2 h auf 2°C abgekühlt, dann 18 h bei 2°C gerührt und innerhalb von 2 h wieder auf 17,5°C aufgeheizt. Dabei wird zunächst eine Druckabnahme gemäß der thermischen Kompression des Gases beobachtet. Tritt während der Unterkühlzeit die Bildung von Gashydratkeimen auf, so verringert sich der gemessene Druck, wobei ein Anstieg des gemessenen Drehmoments und ein leichter Anstieg der Temperatur zu beobachten ist. Weiteres Wachstum und zunehmende Agglomeration der Hydratkeime führen ohne Inhibitor schnell zu einem weiteren Anstieg des Drehmomentes. Beim Erwärmen des Gemisches zerfallen die Gashydrate, so dass man den Anfangszustand der Versuchsreihe erreicht.

Als Maß für die inhibierende Wirkung der erfindungsgemäßen Verbindungen wird die Zeit vom Erreichen der Minimaltemperatur von 2°C bis zur ersten Gasaufnahme (T_{ind}) bzw. die Zeit bis zum Anstieg des Drehmomentes (T_{agg}) benutzt. Lange Induktionszeiten bzw. Agglomerationszeiten weisen auf eine Wirkung als kinetischer Inhibitor hin. Das im Autoklaven gemessene Drehmoment dient dagegen als Größe für die Agglomeration der Hydratkristalle. Der gemessene Druckverlust (Δ p) lässt einen direkten Schluss auf die Menge gebildeter Hydratkristalle zu. Bei einem guten Antiagglomerat ist das Drehmoment, das sich nach Bildung von Gashydraten aufbaut, gegenüber dem Blindwert deutlich verringert. Im Idealfall bilden sich schneeartige, feine Hydratkristalle in der Kondensatphase, die sich nicht zusammenlagern und somit nicht zum Verstopfen der zum Gastransport und zur Gasförderung dienenden Installationen führen.

### Testergebnisse

Zusammensetzung des verwendeten Erdgases:
- Gas 1:: Methan 79,3 %, Ethan 10,8 %, Propan 4,8 %, Butan 1,9 %, Kohlendioxid 1,4 %, Stickstoff 1,8 %. Unterkühlung unter die Gleichgewichtstemperatur der Hydratbildung bei 50 bar: 12°C.
- Gas 2:: Methan 92,1 %, Ethan 3,5 %, Propan 0,8 %, Butan 0,7 %, Kohlendioxid 0,6 %, Stickstoff 2,3 %. Unterkühlung unter die Gleichgewichtstemperatur der Hydratbildung bei 50 bar: 7°C, Unterkühlung bei 100 bar: 12°C.

Um die Wirkung als Agglomeratinhibitoren zu prüfen, wurde im oben verwendeten Testautoklaven Wasser und Testbenzin vorgelegt (20 % des Volumens im Verhältnis 1:2) und bezogen auf die Wasserphase 5000 ppm des jeweiligen Additivs zugegeben. Bei einem Autoklavendruck von 90 bar unter Verwendung von Gas 1 und einer Rührgeschwindigkeit von 5000 U/min wurde die Temperatur von anfangs 17,5°C innerhalb 2 Stunden auf 2°C abgekühlt, dann 25 Stunden bei 2°C gerührt und wieder aufgewärmt. Dabei wurde der Druckverlust durch die Hydratbildung und das dabei auftretende Drehmoment am Rührer gemessen, das ein Maß für die Agglomeration der Gashydrate ist.

**Tabelle 3: (Test als Antiagglomerant)**

| Beispiel | Gashydratinhibitor | Druckverlust Δ p (bar) | Temperaturanstieg ΔT (K) | Drehmoment Mₘₐₓ (Ncm) |
|---|---|---|---|---|
| Blindwert | - | > 40 | > 8 | 15,9 |
| 13 | Quat aus Beispiel 1 | 15,2 | 1,1 | 4,9 |
| 18 | Quat aus Beispiel 6 | 10,3 | 0,5 | 0,5 |
| 23 | Quat aus Beispiel 11 | 14,7 | 1,0 | 1,0 |
| 24 | Quat aus Beispiel 12 | 13,9 | 1,0 | 0,8 |
| Vergleich 3 | TBAB | 21,5 | 1,0 | 1,5 |
| Vergleich 4 | TBAB | 15,0 | 1,0 | 1,2 |

Als Vergleichssubstanzen 3 und 4 wurden zwei kommerziell erhältliche Antiagglomerant- Inhibitoren auf Basis Tetrabutylammoniumbromid (TBAB) herangezogen.

Wie aus diesen Beispielen zu ersehen ist, waren die gemessenen Drehmomente im Vergleich zum Blindwert trotz heftiger Hydratbildung stark reduziert. Dies spricht für eine deutliche agglomeratinhibierende Wirkung der erfindungsgemäßen Produkte.

Um die Wirkung als Additive für kinetische Inhibitoren zu prüfen, wurde im oben verwendeten Testautoklaven 5000 ppm des jeweiligen Additivs bezogen auf die Wasserphase zugegeben und bei unterschiedlichen Drücken unter Verwendung der Gase 1 bzw. 2 abgekühlt. Nach Erreichen der Minimaltemperatur von 2°C wurde die Zeit bis zur ersten Gasaufnahme (T_{ind}) aufgezeichnet. Der gemessene Druckverlust (Δ p) und der Temperaturanstieg Δ T (K) erlaubt den direkten Schluss auf die Menge gebildeter Hydratkristalle.

**Tabelle 4: (Test als kinetische Inhibitoren)**

| Beispiel | Inhibitor | Gas | Druck p (bar) | T_{ind} | Druckverlust Δ p (bar) | Temperaturanstieg Δ T (K) |
|---|---|---|---|---|---|---|
| Blindwert | - | 1 | 50 | 0 | > 40 | > 1,5 |
| Blindwert | - | 2 | 100 | 0 | > 40 | > 1,5 |
| 25 | Quat aus Beispiel 1 | 1 | 50 | 8,0 h | 7,2 | 0,4 |
| 25 | Quat aus Beispiel 1 | 2 | 100 | < 5 min | 10,8 | 0,4 |
| 26 | Quat aus Beispiel 6 | 1 | 50 | 6,5 h | 9,5 | 0,1 |
| 26 | Quat aus Beispiel 6 | 2 | 100 | < 5 min | 10,1 | 0,2 |
| Vergleich 5 | PVCap | 1 | 50 | < 5 min | 10 | 0,4 |
| Vergleich 5 | PVCap | 2 | 100 | < 5 min | 6 | 0,1 |

Als Vergleichssubstanz 5 wurde eine Lösung von Polyvinylcaprolactam (PVCap) in Butylglykol herangezogen, Molekulargewicht 5000 g/mol.

Wie aus den obigen Testresultaten zu erkennen ist, wirken die erfindungsgemäßen Produkte als synergistische Komponente von kinetische Hydratinhibitoren und zeigen eine deutliche Verbesserung gegenüber dem Stand der Technik. Sie können daher zur Steigerung (Synergieeffekt) der Leistungsfähigkeit von Inhibitoren des Standes der Technik verwendet werden.

## Patentansprüche

1. Verwendung von Verbindungen der Formel (1) 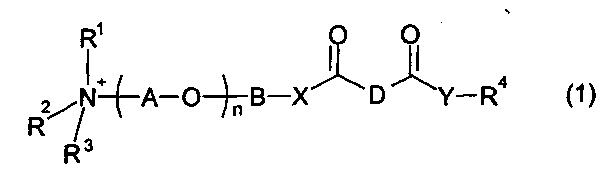 worin
R¹, R² unabhängig voneinander C₁- bis C₂₂-Alkyl, C₂- bis C₂₂-Alkenyl, C₆- bis C₃₀-Aryl- oder C₇- bis C₃₀-Alkylaryl,
R³ C₁- bis C₂₂- Alkyl, C₂- bis C₂₂- Alkenyl, C₆- bis C₃₀-Aryl- oder C₇- bis C₃₀-Alkylaryl, -CHR⁵-COO⁻ oder -O⁻,
R⁴ M, Wasserstoff oder einen gegebenenfalls Heteroatome enthaltenden organischen Rest mit 1 bis 100 Kohlenstoffatomen,
A eine C₂- bis C₄-Alkylengruppe,
B eine C₁- bis C₁₀-Alkylengruppe,
D einen gegebenenfalls Heteroatome enthaltenden organischen Rest mit 1 bis 600 Kohlenstoffatomen,
X, Y unabhängig voneinander O oder NR⁶,
R⁵, R⁶ unabhängig voneinander Wasserstoff, C₁- bis C₂₂-Alkyl, C₂- bis C₂₂-Alkenyl, C₆- bis C₃₀-Aryl- oder C₇- bis C₃₀-Alkylaryl, und
M ein Kation
n eine Zahl von 1 bis 30
bedeuten, als Korrosionsinhibitoren und Gashydratinhibitoren.

2. Verwendung nach Anspruch 1, wobei A für eine Ethylen- oder Propylengruppe steht.

3. Verwendung nach Anspruch 1 und/oder 2, wobei B für eine C₂- bis C₄-Alkylengruppe steht.

4. Verwendung nach einem oder mehreren der Ansprüche 1 bis 3, wobei R¹ und R² unabhängig voneinander für eine Alkyl- oder Alkenylgruppe von 2 bis 14 Kohlenstoffatomen stehen.

5. Verwendung nach einem oder mehreren der Ansprüche 1 bis 4, wobei R³ für eine Alkyl- oder Alkenylgruppe mit 1 bis 12 Kohlenstoffatomen steht.

6. Verwendung nach einem oder mehreren der Ansprüche 1 bis 5, wobei R⁵ und R⁶ für Wasserstoff stehen.

7. Verwendung nach einem oder mehreren der Ansprüche 1 bis 6, wobei n für eine Zahl zwischen 1 und 10 steht.

8. Verwendung nach einem oder mehreren der Ansprüche 1 bis 7, wobei R⁴ für einen Rest der Formel (2) steht 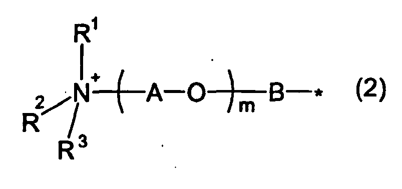 worin R¹, R², R³, A und B die in Anspruch 1 angegebenen Bedeutungen haben, und m unabhängig von n für eine Zahl zwischen 0 und 30 steht.

9. Verwendung nach einem oder mehreren der Ansprüche 1 bis 8, wobei D eine C₂- bis C₅₀-Alkylen- oder C₂- bis C₅₀-Alkenylen-Gruppe ist.

10. Verwendung nach einem oder mehreren der Ansprüche 1 bis 8, wobei D von substituierten Bernsteinsäurederivaten mit 10 bis 100 Kohlenstoffatomen abgeleitet ist.

11. Verwendung nach einem oder mehreren der Ansprüche 1 bis 8, wobei D für einen Rest der Formel (3) steht 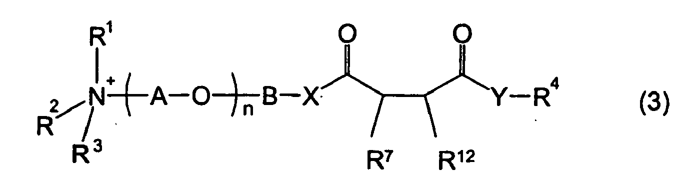 worin
R⁷ und R¹² entweder Wasserstoff oder einen C₂- bis C₁₀₀-Alkyl- oder C₂- bis C₁₀₀-Alkenyl-Rest, der als Oligomer von C₂- bis C₈-Alkenen erhältlich ist und geradkettig oder verzweigt sein kann bedeuten, mit der Maßgabe, dass genau einer der Reste R⁷ und R¹² für Wasserstoff steht, und R¹, R², R³, R⁴, A, B, X, Y und n die in Anspruch 1 angegebenen Bedeutungen aufweisen.

12. Verbindungen der Formel (1), 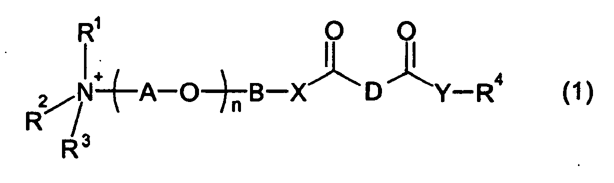 worin
R¹, R² unabhängig voneinander C₁- bis C₂₂-Alkyl, C₂- bis C₂₂-Alkenyl, C₆- bis C₃₀-Aryl- oder C₇₋ bis C₃₀-Alkylaryl,
R³ C₁- bis C₂₂- Alkyl, C₂- bis C₂₂- Alkenyl, C₆- bis C₃₀-Aryl- oder C₇- bis C₃₀-Alkylaryl, -CHR⁵-COO⁻ oder -O⁻,
R⁴ M, Wasserstoff oder einen gegebenenfalls Heteroatome enthaltenden organischen Rest mit 1 bis 100 Kohlenstoffatomen,
A eine C₂- bis C₄-Alkylengruppe,
B eine C₁- bis C₁₀-Alkylengruppe,
D einen gegebenenfalls Heteroatome enthaltenden organischen Rest mit 1 bis 600 Kohlenstoffatomen,
X, Y unabhängig voneinander O oder NR⁶,
R⁵, R⁶ unabhängig voneinander Wasserstoff, C₁- bis C₂₂-Alkyl, C₂- bis C₂₂-Alkenyl, C₆- bis C₃₀-Aryl- oder C₇- bis C₃₀-Alkylaryl, und
M ein Kation
n eine Zahl von 1 bis 30 bedeuten.

## Claims

1. The use of compounds of the formula (1) 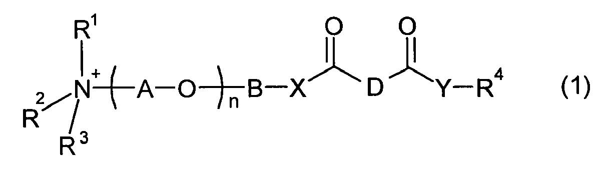 where
R¹, R² are each independently C₁- to C₂₂-alkyl, C₂- to C₂₂-alkenyl, C₆- to C₃₀-aryl or C₇- to C₃₀-alkylaryl,
R³ is C₁- to C₂₂-alkyl, C₂- to C₂₂-alkenyl, C₆- to C₃₀-aryl or C₇- to C₃₀-alkylaryl, -CHR⁵-COO⁻ or -O⁻,
R⁴ is M, hydrogen or an organic radical which optionally contains heteroatoms and has from 1 to 100 carbon atoms,
A is a C₂- to C₄-alkylene group,
B is a C₁- to C₁₀-alkylene group,
D is an organic radical which optionally contains heteroatoms and has from 1 to 600 carbon atoms,
X, Y are each independently O or NR⁶,
R⁵, R⁶ are each independently hydrogen, C₁- to C₂₂-alkyl, C₂- to C₂₂-alkenyl, C₆- to C₃₀-aryl or C₇- to C₃₀-alkylaryl, and
M is a cation
n is a number from 1 to 30
as corrosion inhibitors and gas hydrate inhibitors.

2. The use as claimed in claim 1, wherein A is an ethylene or propylene group.

3. The use as claimed in claim 1 and/or 2, wherein B is a C₂- to C₄-alkylene group.

4. The use as claimed in one or more of claims 1 to 3, wherein R¹ and R² are each independently an alkyl or alkenyl group of from 2 to 14 carbon atoms.

5. The use as claimed in one or more of claims 1 to 4, wherein R³ is an alkyl or alkenyl group having from 1 to 12 carbon atoms.

6. The use as claimed in one or more of claims 1 to 5, wherein R⁵ and R⁶ are hydrogen.

7. The use as claimed in one or more of claims 1 to 6, wherein n is a number in the range from 1 to 10.

8. The use as claimed in one or more of claims 1 to 7, wherein R⁴ is a radical of the formula (2) 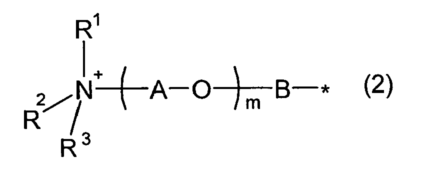 where R¹, R², R³, A and B are each as defined in claim 1, and m, independently of n, is a number in the range from 0 to 30.

9. The use as claimed in one or more of claims 1 to 8, wherein D is a C₂- to C₅₀-alkylene or C₂- to C₅₀-alkenylene group.

10. The use as claimed in one or more of claims 1 to 8, wherein D is derived from substituted succinic acid derivatives having from 10 to 100 carbon atoms.

11. The use as claimed in one or more of claims 1 to 8, wherein D is a radical of the formula (3) 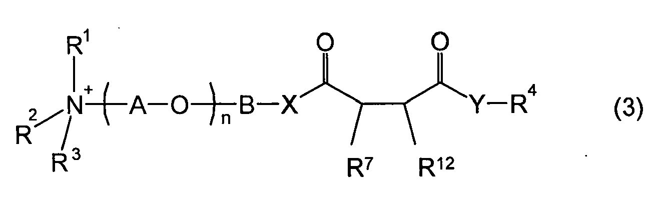 where
R⁷ and R¹² are each either hydrogen or a C₂- to C₁₀₀-alkyl or C₂- to C₁₀₀-alkenyl radical which is obtainable as an oligomer of C₂- to C₈-alkenes and may be straight-chain or branched, with the proviso that exactly one of the R⁷ and R¹² radicals is hydrogen, and R¹, R², R³, R⁴, A, B, X, Y and n are each as defined in claim 1.

12. A compound of the formula (1) 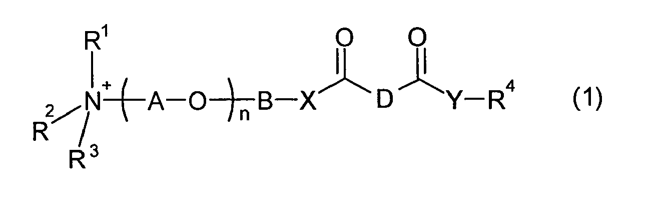 where
R¹, R² are each independently C₁- to C₂₂-alkyl, C₂- to C₂₂-alkenyl, C₆- to C₃₀-aryl or C₇- to C₃₀-alkylaryl,
R³ is C₁- to C₂₂-alkyl, C₂- to C₂₂-alkenyl, C₆- to C₃₀-aryl or C₇- to C₃₀-alkylaryl, -CHR⁵-COO⁻ or -O⁻,
R⁴ is M, hydrogen or an organic radical which optionally contains heteroatoms and has from 1 to 100 carbon atoms,
A is a C₂- to C₄-alkylene group,
B is a C₁- to C₁₀-alkylene group,
D is an organic radical which optionally contains heteroatoms and has from 1 to 600 carbon atoms,
X, Y are each independently O or NR⁶,
R⁵, R⁶ are each independently hydrogen, C₁- to C₂₂-alkyl, C₂- to C₂₂-alkenyl, C₆- to C₃₀-aryl or C₇- to C₃₀-alkylaryl, and
M is a cation
n is a number from 1 to 30.

## Revendications

1. Utilisation de composés de formule (1) 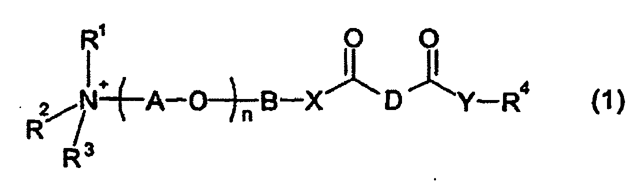 dans laquelle
R¹ et R² représentent, indépendamment l'un de l'autre, un groupe alkyle en C₁ à C₂₂, un groupe alcényle en C₂ à C₂₂, un groupe aryle en C₆ à C₃₀ ou un groupe alkylaryle en C₇ à C₃₀,
R³ représente un groupe alkyle en C₁ à C₂₂, un groupe alcényle en C₂ à C₂₂, un groupe aryle en C₆ à C₃₀ ou un groupe alkylaryle en C₇ à C₃₀, un groupe -CHR⁵-COO⁻ ou un groupe -O⁻,
R⁴ représente M, un atome d'hydrogène ou un radical organique renfermant éventuellement des hétéroatomes, ayant de 1 à 100 atomes de carbone,
A représente un groupe alkylène en C₂ à C₄,
B représente un groupe alkylène en C₁ à C₁₀,
D représente un radical organique renfermant éventuellement des hétéroatomes, ayant de 1 à 600 atomes de carbone,
X et Y représentent, indépendamment l'un de l'autre, un atome d'oxygène ou un groupe NR⁶,
R⁵ et R⁶ représentent, indépendamment l'un de l'autre, un atome d'hydrogène, un groupe alkyle en C₁ à C₂₂, un groupe alcényle en C₂ à C₂₂, un groupe aryle en C₆ à C₃₀ ou un groupe alkylaryle en C₇ à C₃₀, et
M représente un cation,
n représente un nombre de 1 à 30,
en tant qu'inhibiteurs de la corrosion et inhibiteurs d'hydrates de gaz.

2. Utilisation selon la revendication 1, dans laquelle A représente un groupe éthylène ou un groupe propylène.

3. Utilisation selon les revendications 1 et/ou 2, dans laquelle B représente un groupe alkylène en C₂ à C₄.

4. Utilisation selon l'une ou plusieurs des revendications 1 à 3, dans laquelle R¹ et R² représentent, indépendamment l'un de l'autre, un groupe alkyle ou un groupe alcényle ayant de 2 à 14 atomes de carbone.

5. Utilisation selon l'une ou plusieurs des revendications 1 à 4, dans laquelle R³ représente un groupe alkyle ou un groupe alcényle ayant de 1 à 12 atomes de carbone.

6. Utilisation selon l'une ou plusieurs des revendications 1 à 5, dans laquelle R⁵ et R⁶ représentent un atome d'hydrogène.

7. Utilisation selon l'une ou plusieurs des revendications 1 à 6, dans laquelle n représente un nombre compris entre 1 et 10.

8. Utilisation selon l'une ou plusieurs des revendications 1 à 7, dans laquelle R⁴ représente un radical de formule (2) 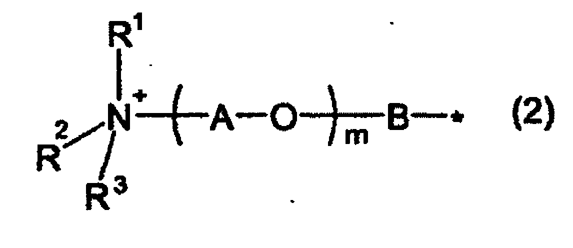 dans laquelle R¹, R², R³, A et B présentent les significations indiquées dans la revendication 1, et m, indépendamment de n, représente un nombre compris entre 0 et 30.

9. Utilisation selon l'une ou plusieurs des revendications 1 à 8, dans laquelle D représente un groupe alkylène en C₂ à C₅₀ ou un groupe alcénylène en C₂ à C₅₀.

10. Utilisation selon l'une ou plusieurs des revendications 1 à 8, dans laquelle D est dérivé de dérivés de l'acide succinique substitués ayant de 10 à 100 atomes de carbone.

11. Utilisation selon l'une ou plusieurs des revendications 1 à 8, dans laquelle D représente un radical de formule (3) 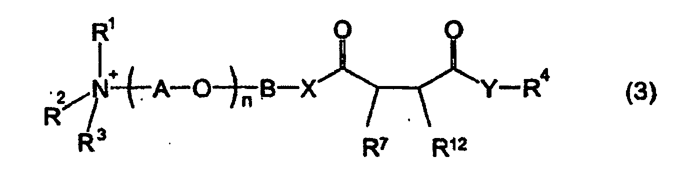 dans laquelle
R⁷ et R¹² représentent soit un atome d'hydrogène, soit un radical alkyle en C₂ à C₁₀₀ ou un radical alcényle en C₂ à C₁₀₀ qui peut être obtenu en tant qu'oligomère d'alcènes en C₂ à C₈ et peut être linéaire ou ramifié, à condition qu'exactement l'un des radicaux R⁷ et R¹² représente un atome d'hydrogène, et R¹, R², R³, R⁴, A, B, X, Y et n présentent les significations indiquées dans la revendication 1.

12. Composés de formule (1), 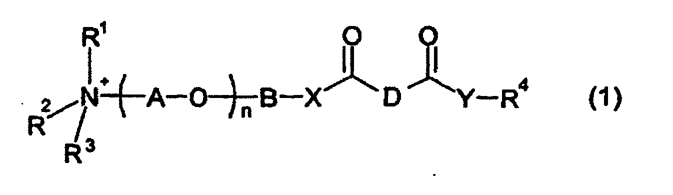 dans laquelle
R¹ et R² représentent, indépendamment l'un de l'autre, un groupe alkyle en C₁ à C₂₂, un groupe alcényle en C₂ à C₂₂, un groupe aryle en C₆ à C₃₀ ou un groupe alkylaryle en C₇ à C₃₀,
R³ représente un groupe alkyle en C₁ à C₂₂, un groupe alcényle en C₂ à C₂₂, un groupe aryle en C₆ à C₃₀ ou un groupe alkylaryle en C₇ à C₃₀, un groupe -CHR⁵-COO⁻ ou un groupe -O⁻,
R⁴ représente M, un atome d'hydrogène ou un radical organique renfermant éventuellement des hétéroatomes, ayant de 1 à 100 atomes de carbone,
A représente un groupe alkylène en C₂ à C₄,
B représente un groupe alkylène en C₁ à C₁₀,
D représente un radical organique renfermant éventuellement des hétéroatomes, ayant de 1 à 600 atomes de carbone,
X et Y représentent, indépendamment l'un de l'autre, un atome d'oxygène ou un groupe NR⁶,
R⁵ et R⁶ représentent, indépendamment l'un de l'autre, un atome d'hydrogène, un groupe alkyle en C₁ à C₂₂, un groupe alcényle en C₂ à C₂₂, un groupe aryle en C₆ à C₃₀ ou un groupe alkylaryle en C₇ à C₃₀, et
M représente un cation,
n représente un nombre de 1 à 30.
